# EUROPEAN PATENT SPECIFICATION

(11) **EP 3 852 824 B1**
(45) Date of publication and mention of the grant of the patent: **13.12.2023**
(21) Application number: 20767872.3
(22) Date of filing: 05.08.2020
(51) Int. Cl.: A61L 27/36, A61L 27/56, A61L 27/58, A61L 31/00, A61L 31/08, A61L 31/14, A61L 27/06, A61L 31/02

(54) **PROCESS FOR COATING IMPLANTS, PLATES, SCREWS OR PROSTHESES MADE OF TITANIUM AND ITS ALLOYS WITH DECELLULARIZED BONE TISSUE (AUTOLOGOUS, HOMOLOGOUS OR HETEROLOGOUS) CONTAINING NATIVE EXTRACELLULAR MATRICES**
VERFAHREN ZUR BESCHICHTUNG VON IMPLANTATEN, PLATTEN, SCHRAUBEN ODER PROTHESEN AUS TITAN UND DESSEN LEGIERUNGEN MIT DEZELLULARISIERTEM KNOCHENGEWEBE (AUTOLOG, HOMOLOG ODER HETEROLOG) MIT NATIVEN EXTRAZELLULÄREN MATRIZEN
PROCÉDÉ DE REVÊTEMENT D'IMPLANTS, DE PLAQUES, DE VIS OU DE PROTHÈSES EN TITANE ET SES ALLIAGES AVEC DES TISSUS OSSEUX DÉCELLULARISÉS (AUTOLOGUES, HOMOLOGUES OU HÉTÉROLOGUES) CONTENANT DES MATRICES EXTRACELLULAIRES NATIVES

(30) Priority: 07.08.2019 IT 201900014316
(43) Date of publication of application: 28.07.2021
(73) Proprietor: Tiss'you Srl, 47895 Domagnano (SM)
(72) Inventor: FATTORI, Paolo, 10040 Rivalta Di Torino (IT)
(74) Representative: Roncuzzi, Davide
(86) International application number: PCT/IB2020/057394
(87) International publication number: WO 2021/024197

(56) References cited:
- WO-A1-2014/024171
- C. LARSSON WEXELL ET AL: "Bone Response to Surface-Modified Titanium Implants: Studies on the Early Tissue Response to Implants with Different Surface Characteristics", INTERNATIONAL JOURNAL OF BIOMATERIALS, vol. 2013, 1 January 2013 (2013-01-01), pages 1-10, XP055744866, ISSN: 1687-8787, DOI: 10.1155/2013/412482
- DEPAULA C A ET AL: "Effects of Hydrogen Peroxide Cleaning Procedures on Bone Graft Osteoinductivity and Mechanical Properties", CELL AND TISSUE BANKING, KLUWER ACADEMIC PUBLISHERS, DO, vol. 6, no. 4, 1 December 2005 (2005-12-01), pages 287-298, XP019234076, ISSN: 1573-6814, DOI: 10.1007/S10561-005-3148-2

## Description

The present invention relates to a process for coating implants, plates, screws or prostheses made of titanium and its alloys with decellularized bone tissue (autologous, homologous or heterologous) containing native extracellular matrices.

Titanium and its alloys are widely used for biomedical purposes for their high resistance to corrosion, excellent biocompatibility, low specific weight and sufficient durability (L Carlsson, T Röstlund, B Albrektsson, T Albrektsson, P I Brånemark, Acta Orthop. Scand. 57 (1986) 285) .

They are used as bone prostheses, plates, screws or dental implants.

Histopathological examination did not reveal mutated cells in the vicinity of titanium implants. An increase of metal elements in the adjacent tissues was observed with spectrochemical analysis; however, no negative clinical effects were detected. There is a darkening of the soft tissue adjacent to the titanium implant, which may be due to the low hardness and poor resistance to abrasion of the unbound material.

Titanium readily forms titanium oxide or complex oxides and hydride compounds. Therefore, it is possible that part of the material removed from the implant is immediately stabilized by the formation of these inert compounds and the tissue seems not to respond to chemically inert titanium oxides.

Titanium oxide is used in creams for treating dermatological conditions. Careful examination of the tissue adjacent to the alloy Ti6Al4V did not reveal any large non-macrophage cells or other signs of inflammation. Therefore, the greatest drawback of these materials derives from their inability to form a chemical bond with the bone tissue, which, in the long term, can lead to loss of the implant or slipping of the bone, with related damage to the tissue adjacent to the area involved.

For this reason, many techniques for activation of the metal surface or coating have been studied.

Coating of the surface is potentially the most promising technique, as it allows binding of substances that interact directly with the tissues in the implant site.

Currently, coating techniques are focused on the use of biocompatible synthetic materials that are able to osseointegrate, such as bioglass and hydroxyapatites.

At the current state of the art, coating methods provide for the use of plasma spray or sintering techniques, forming a uniform surface on the metal implant.

However, the capacity of the aforesaid materials to bind with the device is poor and production techniques are very complex and costly.

Moreover, the process temperatures do not allow the use of organic materials, such as bone tissue (autologous, homologous or heterologous), which would improve the osseointegration of the material due to the presence of the extracellular matrix, which can be defined as containing type I collagen, glucosaminoglycans and the complex of proteins functional for osteoinduction and osteogenesis.

Today, coating with bone tissue powder or collagen is conducted through adhesion by means of weak interaction forces (Van der Waals or ion interactions), activated by lyophilization processes (e.g. WO 2014/024171). The material is therefore weakly bound with the risk of loss during the implantation step.

Some researchers have developed surface activation and/or coating methods via the formation of a titanium gel (M. Karthega, S. Nagarajan, N. Rajendran*, "In vitro studies of hydrogen peroxide treated titanium for biomedical applications" Electrochimica Acta, 55 (2010),2201-2209) using hydrogen peroxide at different concentrations and temperatures. However, to date this technique has been used to coat implants with metal ions, capable of increasing the compatibility of the material (e.g. US 5,855,612), but not its osseointegration.

The process of the present invention is based on the activation of decellularized and deantigenated mammalian bone tissue, in which however the extracellular matrix is present in its native form, as it is not denatured by the process itself.

In fact, the subject matter of the present invention is the process for incorporating decellularized and deantigenated bone tissue on the surfaces of implants, plates, screws or prostheses made of titanium or its alloys, comprising preparing the bone tissue according to known methods to obtain a powder that is suspended in an aqueous solution at 10-35% by volume of hydrogen peroxide, at a temperature ranging from 10 to 30°C for a contact time ranging from 10 minutes to 4 hours, in a ratio of 1:1 by weight, and characterized in that the titanium metal substrate is subsequently introduced into this solution for a maximum time of 5 minutes.

The decellularized and deantigenated tissue used in the present invention is advantageously produced by harvesting mammalian bone sections, as by-product in Class 3 of the food industry, treated both with one of the processes already known to those skilled in the art (e.g. Patent No. IT0001413858, Patent No. DE3617896A1) and with the transesterification process of the lipid components forming the subject matter of International patent application WO 2020/058813 (extension of Italian patent application no. 102018000008649) filed by the present Applicant, which is able to eliminate the antigenic cells and components, leaving the extracellular matrix unchanged (Collagens, Glucosaminoglycans GAGs and the complex of proteins functional for osteoinduction and osteogenesis). This patent application provides for the treatment of mammalian connective tissue in the presence of:
- a primary alcohol R-OH, wherein R is a linear alkyl group C1-C4,
- a hydroxide of an alkaline or alkaline earth metal;
- a water scavenger agent.

The water scavenger agent can be chosen in the group consisting of drying agents; in a variable concentration ranging from 1 to 10% by weight; or the water scavenger agent can be chosen in the group consisting of acetals of general formula(I) 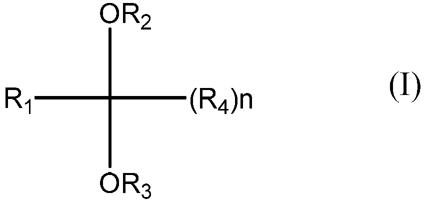 wherein
- R₁: is hydrogen or an alkyl group C1-C4;
- R₂: is an alkyl group C1-C4;
- R₃: is an alkyl group C1-C4;
- R₄: is H, or an alkyl group C1-C4 or is an alkoxy group OR₅;
- R₅: is an alkyl group C1-C4;
- n: varies between 1 and 2; with the condition that R₁ and R₄ are not simultaneously hydrogen; and in a concentration ranging from 1 to 50 mM.

Preferably, the alcohol used is methanol or ethanol; the hydroxide of an alkaline or alkaline earth metal is potassium hydroxide; the water scavenger agent is sodium sulphate or 2,2-Dimethoxypropane. The process is conducted at a temperature ranging from 40° to 60°C, at a pH ranging from 8.0 to 10.0 and at a pressure ranging from 5 kPa to 15 kPa (50 to 150 mbar). This process is illustrated in detail in the Experimental Example below.

After this treatment the tissue is lyophilized and micronized in the form of powder or granules of a size ranging from 1 um to 20 um, produced using a rotor mill followed by an ultra centrifugal mill (SR300 and ZM 200, Retsch) and sieving the powders with a sieve shaker (AS 200, Retsch). The powder is then treated with an aqueous solution of hydrogen peroxide at a concentration ranging from 10 to 35% by volume, suspending the micronized bone tissue in the activating solution in a ratio of 1:1 by weight.

The activation temperature ranges from 10 to 30°C, preferably from 15 to 25°C, for a contact time ranging from 10 minutes to 4 hours, preferably from 30 minutes to 3 hours, even more preferably from 1 hour to 2 and a half hours.

At the end of activation, the bone tissue is deposited on the surface of the implant by immersing the implant itself in the activated solution, for a maximum time of 5 minutes. This passage forms the characteristic element of the present invention, as none of the documents cited provide for the simultaneous presence of the bone powder and of the metal implant in the aqueous solution of hydrogen peroxide. It is deemed, without referring to a particular scientific theory, that this passage is decisive in forming covalent, hence more lasting, bonds that allow the titanium implant to bind to the suitably prepared bone tissue and thus solve the problems cited above.

The resulting device is extracted and placed in a fan oven at a temperature ranging from 40 to 60°C for a maximum of 2 hours.

The thickness of the coating ranges from 10 to 100 um. With the process of the present invention it is thus possible to prepare titanium implants that are
- osteoconductive;
- able to osseointegrate;
- biocompatible.

### Example 1

10 g of equine bone tissue, harvested from equine humerus, by-product in Class 3 of the food industry, reared and slaughtered in European Union countries according to rearing criteria, ante-mortem and post-mortem examination in force at Community level, is decellularized according to the process of forming the subject matter of Italian patent no. 102018000008649, by immersing the tissue for 15 hours in a solution of methanol, KOH 0.6%, 2,2-Dimethoxypropane 2 mM and under stirring (100 rpm) at 60°C at a pressure of 10 kPa (100 mbar).

At the end of the reaction the tissue is washed with osmotic water (electrical conductivity < 12 µs/cm) at 60°C until complete removal of the reaction products and reagents.

The tissue is then lyophilized using a LYOBETA 5PS lyophilizer, at the following conditions: -30°C, primary drying 0°C with slope 10°C/h and secondary drying 20°C with slope 10°C/h, until obtaining a residual humidity ranging from 0.1 to 1% (residual humidity is measured using a Sartorius MA 35 moisture analyzer).

The product thus obtained is micronized using in sequence a rotor mill, an ultra centrifugal mill and a sieve shaker, respectively SR 300, ZM 200 and AS 200 Retsch.

The powder thus obtained is immersed in a solution of Hydrogen peroxide 30% in a ratio of 1:1 by weight.

The solution is kept under stirring for 2 hours at 20°C. At the end of the reaction, a screw 48 mm in length made of Titanium IV is immersed in the solution obtained for 5 minutes and placed in a fan oven at 60°C for 1 hour.

The screw is then washed with demineralized water and left to dry.

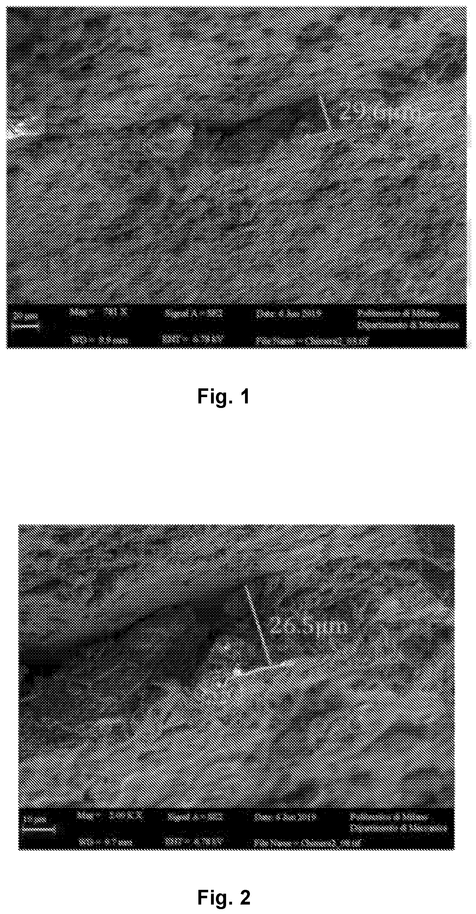

The surface of the screw is thus coated for a thickness of around 30 um, as shown by the SEM photographs (Figures 1 and 2).

Measurements were performed at the laboratories of the Department of Mechanics of Milan Polytechnic, using a SEM Zeiss Sigma 500 microscope.

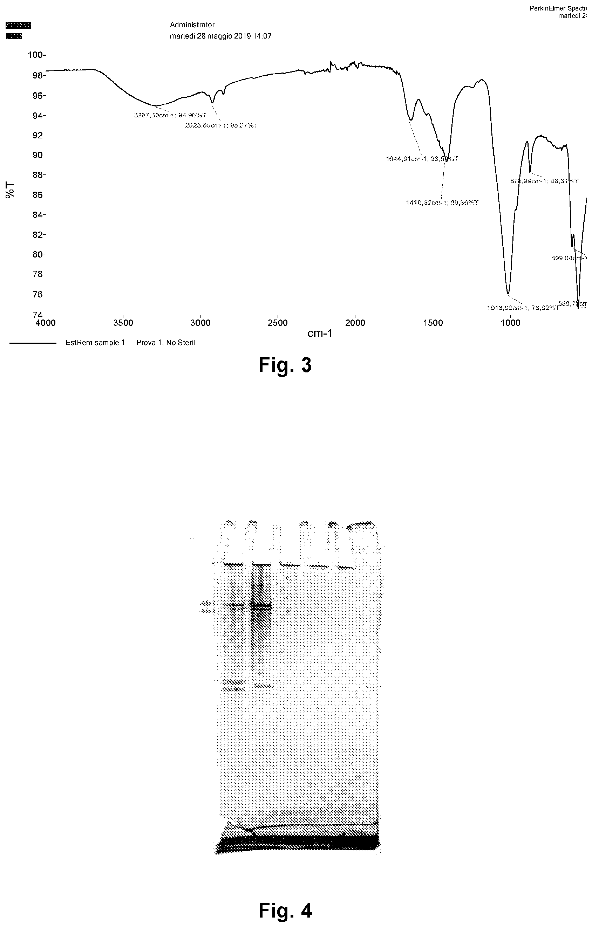

The coating thus obtained is carbonate hydroxyapatite, with the presence of proteins, as shown by the FT analysis (Figure 3), using a PerkinElmer Spectrum Two spectroscope, equipped with ATR module, in which the bands ascribable to the phosphates (1013 cm-1), carbonates (1410 cm-1) and to the resonance of the amide bond I of the proteins (1644 cm-1) can be seen.

The presence of hydroxyapatite, also after repeated rinses in hypotonic and hypertonic solutions and in the presence of surfactants, shows the formation of covalent bonds. The presence of the alpha 1 and alpha 2 chains of bone collagen were highlighted by means of SDS-Page electrophoresis (Figure 4), after demineralization of the powder bound to the surface of the implant, neutralization of the demineralization solution, denaturation in LDS Sample Buffer (NuPage Invitrogen, Life Technologies) and Sample Reducing Agent (Bolt, Novex, Life Technologies), loading of the sample on Gel NuPAGE 4-12% Bis-Tris (Invitrogen, Life Technologies), staining with SYPRO Ruby Protein Gel stain (Lonza) and acquisition of the gel by means of iBright FL1000 (Invitrogen, Life Technologies), using PureCol-S type I Collagen (Sigma-Aldrich) as a reference standard.

## Claims

1. A process for incorporating decellularized and deantigenated bone tissue on the surface of implants, plates, screws or prostheses made of titanium and its alloys, comprising preparing the bone tissue according to known methods until obtaining a powder that is suspended in an aqueous solution at 10-35% by volume of hydrogen peroxide, at a temperature ranging from 10 to 30°C, for a contact time ranging from 10 minutes to 4 hours, in a ratio of 1:1 by weight and **characterized in that** the titanium metal substrate is subsequently introduced into this solution for a maximum time of 5 minutes.

2. The process according to claim 1, which incorporates micronized bone tissue with dimensions ranging from 1 to 100 microns.

3. The process according to claims 1 to 2, comprising the following steps:
• washing the bone tissue in the presence of a primary alcohol R-OH, wherein R is a linear alkyl group C1-C4, a hydroxide of an alkaline or alkaline earth metal; a water scavenger agent; and
• subsequent treatment with known methods until obtaining a bone powder that is suspended in an aqueous solution at 10-35% by volume of hydrogen peroxide, at a temperature ranging from 10 to 30°C, for a contact time ranging from 10 minutes to 4 hours, in a ratio of 1:1 by weight and the titanium metal substrate is subsequently introduced into this solution for a maximum time of 5 minutes.

4. The process according to claim 3 wherein in the step of washing the bone tissue the alcohol used is methanol or ethanol; the hydroxide of alkaline or alkaline earth metal is potassium hydroxide; the water scavenger agent is sodium sulphate or 2,2- Dimethoxypropane; the process is conducted at a temperature ranging from 40° to 60°C, at a pH from 8.0 to 10.0 and at a pressure ranging from 5kPa to 15 kPa (50 to 150 mbar).

5. An implant, plate, screw or prostheses made of titanium or its alloys obtainable by means of the process of claims 1 to 4.

## Patentansprüche

1. Ein Verfahren zum Inkorporieren von dezellularisiertem und deantigeniertem Knochengewebe auf der Oberfläche von Implantaten, Platten, Schrauben oder Prothesen, die aus Titan und seinen Legierungen hergestellt sind, beinhaltend das Vorbereiten des Knochengewebes nach bekannten Methoden bis zum Erhalten eines Pulvers, das in einer wässrigen Lösung mit 10-35 Vol.-% Wasserstoffperoxid bei einer Temperatur in einem Bereich von 10 bis 30 °C während einer Kontaktzeit in einem Bereich von 10 Minuten bis 4 Stunden in einem Verhältnis von 1 : 1 nach Gewicht suspendiert ist, und **dadurch gekennzeichnet, dass** das Titanmetallsubstrat anschließend für eine maximale Zeit von 5 Minuten in diese Lösung eingebracht wird.

2. Verfahren gemäß Anspruch 1, bei dem mikronisiertes Knochengewebe mit einer Größe in einem Bereich von 1 bis 100 Mikrometern inkorporiert wird.

3. Verfahren gemäß den Ansprüchen 1 bis 2, das die folgenden Schritte beinhaltet:
• Waschen des Knochengewebes in Gegenwart eines primären Alkohols R-OH, wobei R eine lineare Alkylgruppe C1-C4 ist, eines Hydroxids eines Alkali- oder Erdalkalimetalls; eines Wasserabfangmittels; und
• anschließendes Behandeln mit bekannten Methoden bis zum Erhalten eines Knochenpulvers, das in einer wässrigen Lösung mit 10-35 Vol.-% Wasserstoffperoxid bei einer Temperatur von 10 bis 30 °C während einer Kontaktzeit von 10 Minuten bis 4 Stunden in einem Verhältnis von 1 : 1 nach Gewicht suspendiert ist und das Titanmetallsubstrat wird anschließend für eine maximale Zeit von 5 Minuten in diese Lösung eingebracht.

4. Verfahren gemäß Anspruch 3, wobei in dem Schritt des Waschens des Knochengewebes der verwendete Alkohol Methanol oder Ethanol ist; das Alkali- oder Erdalkalihydroxid Kaliumhydroxid ist; das Wasserabfangmittel Natriumsulfat oder 2,2-Dimethoxypropan ist; das Verfahren bei einer Temperatur in einem Bereich von 40 °C bis 60 °C, bei einem pH-Wert von 8,0 bis 10,0 und bei einem Druck in einem Bereich von 5 kPa bis 15 kPa (50 bis 150 mbar) durchgeführt wird.

5. Ein Implantat, eine Platte, eine Schraube oder eine Prothese, das/die aus Titan oder seinen Legierungen hergestellt ist, das/die mittels des Verfahrens gemäß den Ansprüchen 1 bis 4 erhältlich ist.

## Revendications

1. Un processus pour l'incorporation de tissu osseux décellularisé et désantigénéisé sur la surface d'implants, de plaques, de vis ou de prothèses faits en titane et ses alliages, comprenant la préparation du tissu osseux selon des procédés connus jusqu'à obtention d'une poudre qui est mise en suspension dans une solution aqueuse à 10 à 35 % en volume de peroxyde d'hydrogène, à une température comprise dans un éventail allant de 10 à 30 °C, pendant un temps de contact compris dans un éventail allant de 10 minutes à 4 heures, dans un rapport de 1/1 en poids et **caractérisé en ce que** le substrat en métal titane est introduit par la suite dans cette solution pendant un temps maximal de 5 minutes.

2. Le processus selon la revendication 1, lequel incorpore du tissu osseux micronisé de dimensions comprises dans l'intervalle allant de 1 à 100 micromètres.

3. Le processus selon les revendications 1 à 2, comprenant les étapes suivantes :
• lavage du tissu osseux en présence d'un alcool primaire R-OH, où R est un groupe alkyle en C1-C4 linéaire, d'un hydroxyde d'un métal alcalinoterreux ou alcalin ; d'un agent capteur d'eau ; et
• traitement subséquent à l'aide de procédés connus jusqu'à obtention d'une poudre d'os qui est mise en suspension dans une solution aqueuse à 10 à 35 % en volume de peroxyde d'hydrogène, à une température comprise dans l'intervalle allant de 10 à 30 °C, pendant un temps de contact compris dans l'intervalle allant de 10 minutes à 4 heures, dans un rapport de 1/1 en poids et le substrat en métal titane est introduit par la suite dans cette solution pendant un temps maximal de 5 minutes.

4. Le processus selon la revendication 3 où, dans l'étape de lavage du tissu osseux, l'alcool utilisé est du méthanol ou de l'éthanol ; l'hydroxyde de métal alcalinoterreux ou alcalin est de l'hydroxyde de potassium ; l'agent capteur d'eau est du sulfate de sodium ou le 2,2-Diméthoxypropane ; le processus est mené à une température comprise dans l'intervalle allant de 40° à 60 °C, à un pH allant de 8,0 à 10,0 et à une pression comprise dans l'intervalle allant de 5 kPa à 15 kPa (de 50 à 150 mbar).

5. Un implant, une plaque, une vis ou une prothèse faits en titane ou ses alliages pouvant être obtenus au moyen du processus des revendications 1 à 4.
